# EUROPEAN PATENT SPECIFICATION

(11) **EP 3 452 830 B1**
(45) Date of publication and mention of the grant of the patent: **05.01.2022**
(21) Application number: 17731801.1
(22) Date of filing: 03.05.2017
(51) Int. Cl.: G01N 33/68

(54) **ASSAY FOR THE DIAGNOSIS OF A NEUROLOGICAL DISEASE**
ASSAY ZUR DIAGNOSE EINER NEUROLOGISCHEN KRANKHEIT
DOSAGE POUR LE DIAGNOSTIC D'UNE MALADIE NEUROLOGIQUE

(30) Priority: 04.05.2016 EP 16001006
(43) Date of publication of application: 13.03.2019
(73) Proprietor: Euroimmun Medizinische Labordiagnostika AG, 23560 Lübeck (DE); ADx NeuroSciences NV, 9052 Gent (BE)
(72) Inventor: VANMECHELEN, Eugeen, 9810 Eke (BE); DE VOS, Ann, 9450 Haaltert (BE); ENGELBORGHS, Sebastiaan, 2660 Hoboken (BE); PETERS, Oliver, 10719 Berlin (DE); SCHIPKE, Carola, 12555 Berlin (DE)
(86) International application number: PCT/EP2017/000549
(87) International publication number: WO 2017/190834

(56) References cited:
- WO-A2-2004/001421
- PING WU ET AL: "Roles of long noncoding RNAs in brain development, functional diversification and neurodegenerative diseases", BRAIN RESEARCH BULLETIN, vol. 97, 1 August 2013 (2013-08-01), pages 69-80, XP055183033, ISSN: 0361-9230, DOI: 10.1016/j.brainresbull.2013.06.001
- TARAWNEH RAWAN ET AL: "Diagnostic and Prognostic Utility of the Synaptic Marker Neurogranin in Alzheimer Disease", JAMA NEUROLOGY,, vol. 73, no. 5, 1 May 2016 (2016-05-01), pages 561-571, XP009191047,
- RIZVI SYED MOHD DANISH ET AL: "Fetzima (levomilnacipran), a drug for major depressive disorder as a dual inhibitor for human serotonin transporters and beta-site amyloid precursor protein cleaving enzyme-1", CNS & NEUROLOGICAL DISORDERS, BENTHAM SCIENCE PUBLISHERS LTD., NL, vol. 13, no. 8, 1 January 2014 (2014-01-01), pages 1427-1431, XP009191064, ISSN: 1871-5273
- POST ET AL: "Toward a Reliable Distinction Between Patients with Mild Cognitive Impairment and Alzheimer-Type Dementia Versus Major Depression", BIOLOGICAL PSYCHIATRY, ELSEVIER SCIENCE, NEW YORK, NY; US, vol. 59, no. 9, 1 May 2006 (2006-05-01), pages 858-862, XP005398369, ISSN: 0006-3223, DOI: 10.1016/J.BIOPSYCH.2005.09.007
- ANN DE VOS ET AL: "The Cerebrospinal Fluid Neurogranin/BACE1 Ratio is a Potential Correlate of Cognitive Decline in Alzheimer's Disease", JOURNAL OF ALZHEIMER'S DISEASE, vol. 53, no. 4, 6 July 2016 (2016-07-06), pages 1523-1538, XP055389766, NL ISSN: 1387-2877, DOI: 10.3233/JAD-160227
- Díez-Guerra F. Javier: "Neurogranin, a link between calcium/calmodulin and protein kinase C signaling in synaptic plasticity", IUBMB Life, vol. 62, no. 8, 21 July 2010 (2010-07-21), pages 597-606, XP055836486, ISSN: 1521-6543, DOI: 10.1002/iub.357

## Description

The present invention relates to a diagnostically useful carrier comprising a means for capturing Neurogranin, a means for capturing BACE1 and preferably a means for capturing one or more additional biomarkers; a kit comprising the diagnostically useful carrier, optionally further comprising a means for detecting Neurogranin, a means for detecting BACE1 and preferably a means for detecting the one or more additional biomarkers; and a method comprising the steps contacting a sample from a subject with a means for capturing Neurogranin, isolating the means for capturing Neurogranin from the sample and contacting a sample from a subject with a means for capturing BACE1 and isolating the means for capturing BACE1 from the sample.

Developing diagnostic systems for neurological diseases is a continuing challenge in biomedical science, not in the least because many neurological symptoms encountered may be accounted for by a huge variety of causes including genetically-inherited diseases, drug abuse, malnutrition, infection, injury, psychiatric illness, immunological defects and cancer.

Since a neurological disease is rarely associated with a unique pattern of clinical symptoms, it is often difficult to provide a reliable diagnosis based on the observation and clinical examination of the patients or based on the medical histories.

The importance of an early diagnosis cannot be overemphasized. Many neurological disorders, most prominently neurodegenerative diseases, such as Alzheimer's disease (AD) and Parkinson's disease (PD), cannot be cured at this moment, but drugs are available that may be used to slow down their progression. The earlier the diagnosis, the better the chances to exploit the spectrum of available drugs to the full benefit of the patient.

In addition, neurodegenerative diseases progress at strikingly different rates in individual patients. While the symptoms deteriorate within several months in some patients, others may go on to live with mild symptoms for years.

Any option to assess the future development may help patients, their medical doctors, relatives and care takers to adjust the treatment and the patients' lifestyles accordingly. This may also be a useful option for patients who are at elevated risk of developing AD or PD or psychiatric conditions such as depression.

Unfortunately, currently available diagnostic biomarkers do not allow for the prediction of the future progression of the disease. In particular, tau and A_{β} are state markers with limited prognostic value regarding future cognitive deterioration. Tangles and plaques are only weakly correlated with cognitive impairment.

The state of the art discloses a range of assays. EP 1 774 023 discloses a method to diagnose the occurrence of brain damage based on the detection of full-length Neurogranin in various types of samples, but does not disclose an assay detecting BACE1 in cerebrospinal fluid CSF in addition.

PING WU ET AL: "Roles of long noncoding RNAs in brain development, functional diversification and neurodegenerative diseases",BRAIN RESEARCH BULLETIN, vol. 97, 1 August 2013 (2013-08-01), pages 69-80 mentions markers relevant in neurodegenerative diseases. BACE1 and Neurogranin are among the mentioned markers.

Barao *et al.* (2013) disclose a sandwich ELISA for the detection of BACE1, but not Neurogranin. However, BACE1 is referred to as a "poor diagnostic marker for AD", with "very small" differences between samples from AD patients and control groups. Clearly, there is the need to develop diagnostic assays that can be used to predict the onset or future course of such diseases.

Therefore, the problem underlying the present invention is to provide a diagnostic assay that may be used to diagnose neurodegenerative diseases, more specifically AD.

In particular, such assay should allow to diagnose patients having specific and/or unspecific symptoms that may be associated both with neurodegenerative and other neurological diseases or psychiatric diseases such as depression.

Another problem underlying the present invention is to provide a diagnostic assay that allows the prediction of the future progression of neurodegenerative diseases, more specifically AD.

Another problem underlying the present invention is to provide an assay that may be used for the diagnosis of psychiatric diseases, more specifically depression.

The problem underlying the present invention is solved by the subject-matter of the attached independent and dependent claims.

In a first aspect, the problem underlying the present invention is solved by a diagnostically useful carrier comprising a means for capturing Neurogranin, a means for capturing BACE1 and preferably a means for capturing one or more additional biomarkers as defined in claim 1.

In a second aspect, the problem underlying the present invention is solved by a kit comprising the diagnostically useful carrier according to the present invention, optionally further comprising a means for detecting Neurogranin, a means for detecting BACE1 and preferably a means for detecting the one or more additional biomarkers as defined in claim 3.

In a preferred embodiment, the means for specifically capturing Neurogranin and the means for specifically capturing BACE1 are spatially separated.

In another preferred embodiment, the diagnostically useful carrier is selected from the group comprising a bead, a test strip, a microtiter plate, a blot, preferably from the group comprising western blot, line blot and dot blot, a glass surface, a slide, a biochip, a membrane, a microarray, an electrophoresis gel and a submicrometer sized particle and is more preferably either
a microtiter plate having a well comprising the means for specifically capturing Neurogranin, having a well comprising the means for specifically capturing BACE1 and optionally having one or more additional wells, each comprising a means for specifically capturing the one or more additional biomarkers or
a bead comprising the means for specifically capturing Neurogranin and a bead comprising the means for specifically capturing BACE1 and, optionally, one or more additional beads, each comprising a means for specifically capturing the one or more additional biomarkers, most preferably said microtiter plate.

In a third aspect, the problem underlying the present invention is solved by a method comprising the steps
(a1) Contacting a sample from a subject with a means for capturing Neurogranin,
(b1) Isolating the means for capturing Neurogranin from the sample and
(a2) Contacting a sample from a subject with a means for capturing BACE1 and
(b2) Isolating the means for capturing BACE1 from the sample as defined in claim 6.

In another preferred embodiment, the method further comprises the steps
(c1) Contacting the means for capturing Neurogranin with a detectable ligand binding to Neurogranin and
(c2) Contacting the means for capturing BACE1 with a detectable ligand binding to BACE1.

In another preferred embodiment, the method further comprises the steps
(d1) Detecting any Neurogranin captured and
(d2) Detecting any BACE1 captured.

In another preferred embodiment, the method further comprises the steps
(a3) Contacting a sample from a subject with a means for capturing one or more additional biomarkers,
(b3) Isolating the means for capturing the one or more additional biomarkers from the sample, and optionally
(c3) Contacting the means for capturing the one or more additional biomarkers with a detectable ligand binding to the one or more additional biomarkers, and optionally
(d3) Detecting any of the one or more additional biomarkers captured In a 4^{th} aspect, the problem underlying the present invention is solved by a diagnostically useful carrier obtainable by the method according to the present invention.

In another preferred embodiment, the one or more additional biomarkers are selected from the group comprising Abeta42 and -40, P-tau, T-tau and neurofilament protein.

In another preferred embodiment, the carrier, kit or method is adapted for detecting Neurogranin, BACE1 and optionally one or more additional biomarkers using a method from the group comprising immunodiffusion techniques; immunoelectrophoretic techniques; light scattering immunoassays; agglutination techniques; labeled immunoassays such as those from the group comprising radiolabeled immunoassay, enzyme immunoassays such as colourimetric assays, chemiluminscence immunoassays, fluorescence immunoassays, nanogold labeling; mass spectrometry; and Surface Plasmon Resonance, preferably enzyme immunoassay.

In another preferred embodiment, the carrier, kit or method is adapted for detecting Neurogranin, BACE1 and optionally one or more additional biomarkers based on a sandwich ELISA.

In a 5^{th} aspect, the problem underlying the present invention is solved by a use of the diagnostically useful carrier or kit according to the present invention for diagnosing a neurological or neurodegenerative disease, preferably Alzheimer's disease (AD) as defined in claim 13.

In another preferred embodiment, the diagnosis aims to predict the rate of future cognitive decline.

In another preferred embodiment, the diagnosis aims to distinguish a neurodegenerative disease, preferably mild AD, and depression with or without cognitive impairment.

The invention relates to a diagnostically useful carrier, which is preferably a solid carrier made from an artificial material such as glass or plastic. This carrier is associated with a means for specifically capturing an antibody in a bodily fluid sample from a subject, preferably a mammalian subject, more preferably a human subject. In a preferred embodiment, the solid carrier is a diagnostic device, more preferably selected from the group comprising a bead, a test strip, a microtiter plate, a blot, preferably from the group comprising western blot, line blot and dot blot, a glass surface, a slide, a biochip, a membrane, a microarray, an electrophoresis gel and a submicrometer sized particle, more preferably either a microtiter plate or a bead, most preferably said microtiter plate. The diagnostic carrier may be a one-piece diagnostic carrier, for example a microtiter plate having more than one well, for example one well comprising a means for capturing Neurogranin and another well comprising a means for capturing BACE1, or it may comprise two or more pieces, each of which may be used for a separate assay. For example, the diagnostic device may comprise a first bead comprising a means for capturing Neurogranin and another bead comprising a means for capturing BACE1.

According to the present invention, the carrier comprises two or more means for specifically capturing a polypeptide, one means for capturing Neurogranin as defined in claim 1 and one means for capturing BACE1, optionally one or more means for capturing one or more additional biomarkers such as one, two, three, four, five, ten, fifty, one hundred or one thousand more means, each for capturing an additional biomarker.

Said means is immobilized on said carrier such that the means is well placed to interact with and bind to the polypeptide to be captured such as Neurogranin, BACE1 or any additional biomarker when contacted with a liquid sample comprising said polypeptide to be captured to the effect that a complex comprising the means and the polypeptide to be captured is formed. The means may be any reagent binding specifically to Neurogranin, BACE1 or any additional biomarker and is preferably selected from the group comprising an antibody, preferably a monoclonal or polyclonal, more preferably a monoclonal antibody, and an aptamer, more preferably an antibody. Calmodulin or a variant thereof may be used as a means binding specifically to Neurogranin in addition to an antibody or an aptamer. The antibody is preferably an isolated and/or recombinant antibody. The person skilled in the art is familiar with methods that may be used to generate such a means.

Preferably, the means for capturing has a binding affinity K_{D} to the molecule to be captured, preferably Neurogranin, BACE1 or any additional biomarker, of less, *i.e.* a value indicating stronger binding, than 1 mM, in order of increasing preference, 0.5 mM, 0.250 mM, 0.100 mM, 50 µM, 25 µM, 10 µM, 5 µM, 1 µM, 0.5 µM, 0.25 µM, 0.1 µM, 50 nM, 25 nM, 10 nM, 1 nM, 100 pM, 50 pM, 25 pM,10 pM, 5pM, 1pM, 0.5pM, 0.25pM, 0.1pM as measured using surface plasmon resonance in PBS at pH7 at 25 °C.

The term "Neurogranin", as used herein, refers to full-length mammalian, preferably human Neurogranin as represented by database entry Uniprot Q92686 or a fragment therof (not claimed), and a C-terminally truncated fragment of said Neurogranin that comprises at least SEQ ID NO 4, and optionally additional N-terminal amino acid residues, but lacks the three C-terminal residues of full-length Neurogranin, more specifically S76, G77 and D78, which truncated form is also referred to as "neurogranin trunc p75" throughout this application (as in the claimed invention).

Neurogranin or the fragment thereof, as detected, may carry one ore more modifications including those selected from the group comprising a N-acetylation such as e.g. N-acetyl-Met1, a phosphorylation such as phospho-Ser36, and a citrullination such as for e.g. Arg68 based on the numbering of SEQ ID NO 1. Any database entry referred to throughout this document refers to the version and sequence in that database as on-line on the date of the application.

In a preferred embodiment, the term "BACE1", as used herein, refers to full-length mammalian, preferably human BACE1 or a fragment thereof, preferably the fragment represented by SEQ ID NO 2. BACE1 or a fragment thereof, as detected, may carry one or more modifications including those selected from the group comprising an acetylation preferably of a lysine side chain selected from the group comprising Lys105, Lys254, Lys258, Lys264, Lys278, Lys279, and Lys286, a glycosylation preferably of an asparagine side chain selected from the group comprising Asn132, Asn151, Asn202 and Asn333, a disulfide bond preferably between a pair of cysteine side chains selected from the group comprising Cys195 and Cys399, Cys257 and Cys422 as well as Cys309 and Cys359. (All of the above amino acid numbering is based on SEQ ID NO 2.).

In a preferred embodiment, the one or more additional biomarkers are biomarkers that may be used to diagnose a neurodegenerative disease, more preferably AD, more preferably an AD symptom selected from psychiatric conditions, such as depression. The one or more additional biomarkers are preferably selected from the group comprising Abeta42 (Aβ₁₋₄₂), preferably represented by SEQ ID NO 5, Abeta40 (Aβ₁₋₄₀), preferably represented by SEQ ID NO 6, Tau as preferably represented by SEQ ID NO 7, either total tau, also referred to as t-tau, or tau comprising at least one phosphorylated sequence motif, also referred to as p-tau, and neurofilament protein, preferably as represented by SEQ ID NO 8. In a preferred embodiment, the term "t-tau", as used herein, comprises the entirety of all tau molecules or fragments thereof. In another preferred embodiment, the term "p-tau", as used herein, comprises the entirety of all phosphorylated tau molecules or fragments thereof.

According to the present invention, a means for detecting the captured polypeptide, such as Neurogranin, BACE1 or the one or more additional biomarkers, is required. In a preferred embodiment, a secondary antibody or aptamer, different or the same as the primary, capturing antibody or aptamer, binding to the captured polypeptide is used. The secondary antibody or aptamer may be associated with a label that is straightforward to detect, for example a fluorescent, radioactive or enzymatically active label, the latter of which may catalyze a chemiluminescent reaction or the generation of a molecule detectable using colourimetry or spectroscopy or another analytical method.

The means for detecting is preferably designed such that it binds the captured polypeptide and the latter may be detected whilst bound. For example, if a primary antibody is used to capture the polypeptide in a sandwich-like fashion, then the secondary antibody binds to another epitope which is still accessible while the polypeptide is bound to the first antibody.

According to the present invention, there is provided a first reaction mixture comprising the diagnostically useful carrier, a sample and a means for capturing Neurogranin, which means is optionally in complex with Neurogranin, and a second reaction mixture, which is in contact with the first reaction mixture or spatially separated, preferably spatially separated, comprising a diagnostically useful carrier, a sample and a means for capturing BACE1, which means is optionally in complex with BACE1. The Neurogranin in complex may be associated with a detectable ligand to Neurogranin. The BACE1 in complex may be associated with a detectable ligand to BACE1.

The inventive teachings provide a kit, preferably for diagnosing neurodegenerative diseases, more preferably for diagnosing AD. Such a kit is a container that comprises specific reagents required to practice the inventive method, in particular the diagnostically useful carrier according to the present invention, optionally one or more solutions required to practice the inventive method in addition, preferably selected from the group comprising sample dilution buffer, washing buffer and buffer comprising a means for detecting any captured polypeptide, such as a secondary antibody. Furthermore, it may comprise instructions detailing how to use the kit and the inventive diagnostically useful carrier for contacting the inventive polypeptide with a bodily fluid sample from a subject, preferably a human subject. Furthermore, the kit may comprise a positive control, for example recombinant Neurogranin and/or BACE1, known to bind to the means for capturing Neurogranin, BACE1 and, optionally, one or more additional biomarkers, and a negative control. Finally, such a kit may comprise one or more standard solutions for preparing a calibration curve, for example a standard solution comprising Neurogranin at a known concentration, a standard solution comprising BACE1 at a known concentration and, optionally, one or more standard solutions, each comprising an additional biomarker at a known concentration.

In a preferred embodiment, the term "detecting", as used herein, means that the presence or absence of the polypeptide to be detected, preferably Neurogranin, BACE1 or the one or more additional biomarkers, may be determined. In a more preferred embodiment, the term refers to an at least semi-quantitative determination, allowing for a rough approximation of the concentration. In a most preferred embodiment, the term refers to determining the concentration of the polypeptide to be detected, either relative or absolute, such that the concentration ratio of Neurogranin and BACE1 and, optionally, the ratio of Neurogranin or BACE1 to one or more additional biomarkers, in the sample may be determined. For example, the unknown concentration of both Neurogranin and BACE1 in a sample may be determined by running an ELISA of the sample to be examined in parallel with known amounts of Neurogranin and of BACE1, respectively, in separate reactions, followed by establishing calibration curves each for Neurogranin and BACE1, based on the known amounts, and determining the first unknown concentration of Neurogranin and BACE1 in the sample. In a preferred embodiment, the method is selected from the group comprising method from the group comprising immunodiffusion techniques, immunoelectrophoretic techniques, light scattering immunoassays, agglutination techniques, labeled immunoassays such as those from the group comprising radiolabeled immunoassay, enzyme immunoassays such as colourimetric assays, chemiluminscence immunoassays, fluorescence immunoassays, nanogold labeling; mass spectrometry, and Surface Plasmon Resonance, preferably enzyme immunoassay. Such methods are described in the state of the art, for example in Fredolini et al. (2016) Immunocapture strategies in translational proteomics, Expt. Rev. Proteomics 13(1), 83-98.

In a preferred embodiment, the means for capturing Neurogranin, BACE1 and, optionally, the one or more additional biomarkers, are spatially separated, *i.e.* separated in different reaction compartments such that there is no transfer of reagents when then concentrations are determined. Alternatively, the reactions may be carried out in a one-pot manner provided there is the possibility to determine the ratio of Neurogranin, BACE1 and, optionally, the one or more additional biomarkers, for example by using different means for detecting not interfering with each other. In another embodiment, the two or more means for capturing may be separated following the capturing reaction, which is carried out in the same vessel, for the separate determination of the concentration of Neurogranin, BACE1 and of the one or more additional biomarkers. In another embodiment, the diagnostically useful carrier comprises two or more means for capturing, but is split up following the capturing reaction and reacts, in two spatially separate reactions, with two more different means for the detection of Neurogranin, BACE1 and, optionally, of one or more additional biomarkers. In a preferred embodiment, the same sample is subsequently or concomitantly subject to reactions for the detection of Neurogranin, BACE1 and, optionally, one or more additional biomarkers.

In a preferred embodiment, the diagnostically useful carrier is a bead, which is preferably a particle to which a means for capturing a biomarker may be attached such that the bead, together with such means, may be separated from a liquid solution, based on its sedimentation properties, more efficiently than the means for capturing the biomarker by itself, for example by decanting, centrifugation, filtrations, preferably decanting. Typically such bead is a bead made of a carbohydrate which is chemically inert in aqueous solution, for example an agarose or sepharose bead. The bead may be a magnetic bead which may comprise iron at a content of 5 to 30 % (weight/weight). The bead may have a size of 0.1 to 10 µm, preferably 0.5 to 5 µm. The diagnostically useful carrier may comprise a bead coated with a means for capturing Neurogranin, preferably an antibody to Neurogranin, and a bead coated with a means for capturing BACE1, preferably an antibody to BACE1, and, optionally one or more beads coated with a means for capturing the one or more additional biomarkers, preferably an antibody binding to the additional biomarker for each bead, and, optionally, with a means for capturing the one or more additional biomarkers, preferably an antibody or aptamer, preferablay antibody to the one or more additional biomarkers.

In another preferred embodiment, the diagnostically useful device is a microtiter plate comprising a well coated with a means for specifically capturing Neurogranin, a well coated with a means for specifically capturing BACE1 and, optionally, one or more wells, each comprising a means for capturing an additional biomarker.

The sample tested according to the present invention is a blood sample, a brain tissue sample or a CSF sample, preferably a CSF sample, more preferably a CSF sample from a live patient, preferably suspected of suffering from or showing symptoms characteristic for a neurodegenerative disease, preferably initial symptoms of a neurodegenerative disease such as AD.

The present invention relates to a method comprising the steps detecting in a sample from a subject Neurogranin, BACE1 and, optionally, one or more additional biomarkers. Such a method may comprise the steps providing a sample from a subject, contacting the sample with the diagnostically useful carrier according to the present invention under conditions compatible with the formation of a complex comprising the diagnostically useful carrier and the polypeptides to be detected such as Neurogranin and BACE1, for example by simply immersing the diagnostically useful carrier with the sample or with diluted sample for a sufficient period of time. Another subsequent step may involve isolating any complex from the remainder of the sample, for example by gently removing sample from the carrier following formation of the complex such that the latter is preserved, for example by decanting. Subsequently, the diagnostically useful carrier may be washed, again under conditions that allow the components of the complex formed to remain associated. Finally, the complex may be detected, either by directly detecting the intact complex, for example by adding a detectable ligand binding to the complex, but not to the means for capturing by itself. Alternatively, the complex may be detected by dissociating the complex following removal of the sample, removing the polypeptide to be detected from the means for capturing and detecting any dissociated polypeptide. The method is preferably an *in vitro* method.

In a preferred embodiment, a detectable ligand is used to detect a polypeptide such as Neurogranin, BACE1 and, optionally, the one or more additional biomarkers. Preferably the ligand may have a binding affinity K_{D} to the polypeptide to be detected of less, *i.e.* a value indicating stronger binding, than 1 mM, in order of increasing preference, 0.5 mM, 0.250 mM, 0.100 mM, 50 µM, 25 µM, 10 µM, 5 µM, 1 µM, 0.5 µM, 0.25 µM, 0.1 µM, 50 nM, 25 nM, 10 nM, 1 nM, 100 pM, 50 pM, 25 pM, 10 pM, 5pM, 1pM, 0.5pM, 0.25pM or 0.1pM as measured using surface plasmon resonance in PBS at pH7 at 25 °C. Most preferably the ligand has binding affinity K_{D} to the polypeptide to be detected of 1 nM or less. In a preferred embodiment, the detectable ligand is preferably selected from the group comprising an antibody, preferably a monoclonal or polyclonal, more preferably a monoclonal antibody, or an aptamer, more preferably an antibody. The antibody is preferably an isolated and/or recombinant antibody. The detectable ligand may be associated with an artificial label, preferably from the group comprising an enzymatically active polypeptide, a radionucleotide, a spin label, a fluorescent label and a chromophor label.

In a preferred embodiment, the polypeptide to be detected, more specifically neurogranin and BACE1 and, optionally, one or more additional biomarkers, is detected using a sandwich ELISA. Briefly, the diagnostically useful carrier, preferably a microtiter plate or bead, comprises a first antibody or aptamer immobilized on the carrier that serves as the means for capturing the polypeptide to be detected. The complex comprising the first antibody or aptamer and the polypeptide to be detected is detected by addition of a second antibody or aptamer which carries a label and binds to an epitope the same or other than the epitope binding the first antibody. Various immunoassays for detecting neurogranin and BACE1 are described in the state of the art, for example in De Vos et al., 2015, Kvartsberg et al., 2015, Thorsell et al, 2010, WO2012/155134 and Barao et al., 2013. In a preferred embodiment, a sandwhich assay is used to detect neurogranin using a first monoclonal antibody as a capture antibody and a second monoclonal antibody as a detection antibody. In a more preferred embodiment the detection antibody binds to a neurogranin epitope closer to the C-terminus, preferably to an epitope of at least five amino acids, preferably at least 10 amino acids comprising SEQ ID NO 3 (AGGGP) than the epitope to which the capture antibody binds. Most preferably both antibodies bind to neurogranin epitopes located in the collagen-lilke domain of neurogranin as represented by SEQ ID NO 4.

In a preferred embodiment, the term "diagnosis", as used herein, refers to any kind of procedure aiming to obtain information instrumental in the assessment of whether a patient suffers from or is likely or more likely than the average or a comparative subject, the latter preferably having similar symptoms, to suffer from a certain disease or condition in the past, at the time of the diagnosis or in the future, to find out how the disease is progressing or is likely to progress in the future or to evaluate the responsiveness of a patient with regard to a certain treatment, for example the administration of suitable drugs slowing down the progress of a neurodegenerative disease. In other words, the term "diagnosis" comprises not only diagnosing, but also prognosticating and/or monitoring the course of a disease or disorder. In a preferred embodiment, the disease or disorder is a neurological disease, more preferably a neurodegenerative disease, more preferably AD, and this may be distinguished from depression with or without cognitive impairment, preferably without cognitive impairment, using the teachings of the present invention. In people aged at least 40, more preferably 50, more preferably 60, more preferably 65, more preferably 70, more preferably 75, more preferably 80 years.

Therefore, the term "diagnosis" does preferably not imply that the diagnostic methods or agents according to the present invention will be definitive and sufficient to finalize the diagnosis on the basis of a single test, let alone parameter. Rather it may refer to a contribution to what is referred to as a "differential diagnosis", *i.e.* a systematic diagnostic procedure considering the likelihood of a range of possible conditions on the basis of certain diagnostic parameters. This may involve diagnosing a disease or condition indirectly by way of ruling out another. The term "diagnosis" may also refer to a method or agent used to choose the most promising treatment regime for a patient. In other words, the method or agent may relate to selecting a treatment regimen for a subject.

According to the present invention, the diagnostic carrier or kit or method may be used to detect Neurogranin and BACE1 in a sample from a subject. In case such a subject is a patient suffering from mild cognitive impairment (MCI) due to Alzheimer's Disease, or from dementia due to Alzheimer's Disease, such as the patients described in the examples of this invention, the ratio of Neurogranin on BACE1 in the CSF of that subject may be used to assess or to monitor the rate of progression of cognitive decline of that individual by means of changed values of Mini-Mental State Examination (MMSE) over time. A MMSE value of 30 indicates a normal, cognitively healthy status. A MMSE value of less than 30, more preferably 25 or less, indicates MCI or dementia due to AD. The ratio of neurogranin on BACE1 in CSF may be used to assess or to monitor the rate of progression of Alzheimer's Disease, with a high ratio indicating a higher likelihood of a faster rate of decline than a lower ratio. An MCI or AD patient may be diagnosed by subjecting a CSF sample to the inventive method, wherein preferably a ratio of Neurogranin and BACE1 below 0.105 in that CSF sample) may indicate a decrease in MMSE of less than 0.42 MMSE units over the next 6 years. Preferably a ratio of Neurogranin and BACE1 between 0.105 and 0.144 may indicate a decrease in MMSE between 1.64 and 1.84 MMSE units over the next 6 years. Preferably a ratio of Neurogranin and BACE1 greater than 0.144 would indicate a decrease in MMSE more than 2.68 MMSE units over the next 6 years. In a preferred embodiment, the term "ratio of Neurogranin and BACE1", as used herein, refers to the concentration in pg/mL of neurogranin in the sample of interest divided by the concenctration in pg/mL of BACE1 in the same sample.

The present invention is further illustrated by the following non-limiting examples, sequences and figures from which further features, embodiments, aspects and advantages of the present invention may be taken.

**Sequences in this application include**

| | |
|---|---|
| SEQ ID NO 1: | Minimum sequence of neurogranin to be detected |
| SEQ ID NO 2: | Sequence of mature BACE1 without the transmembrane domain and the cytoplasmic tail to be detected (Based on uniprot sequence P56817 from position 22 to 460) |
| SEQ ID NO 3: | C-terminal neurogranin epitope (AGGGP) |
| SEQ ID NO 4: | Collagen-like domain of neurogranin |
| SEQ ID NO 5: | Abeta42 |
| SEQ ID NO 6: | Abeta40 |
| SEQ ID NO 7: | Tau |
| SEQ ID NO 8: | Neurofilament protein |

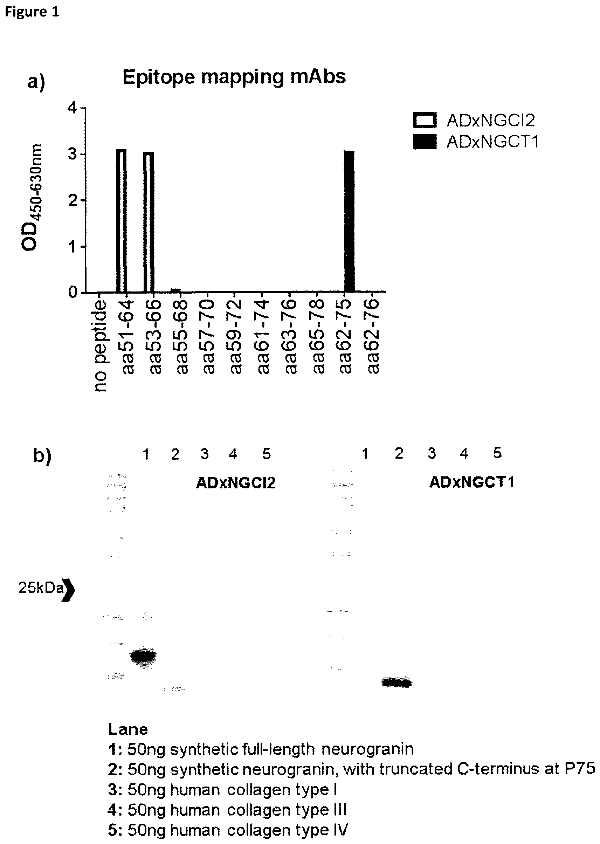

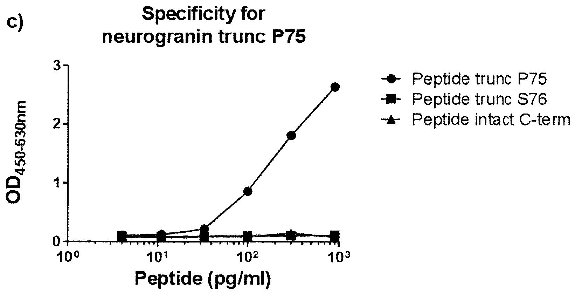

**Fig. 1** shows
   (**a**) epitope mapping of both mAbs ADxNGCI2 and ADxNGCT1, performed with overlapping synthetic peptides ranging from R51 to D78.
   (**b**) specificity of the mAbs ADxNGCI2 and ADxNGCT1 was evaluated by Western blot analysis were both synthetic full-length neurogranin as well as a synthetic neurogranin peptide that is truncated at P75 were included in the gel electrophoresis, next to human collagen type I, III and IV. ADxNGCI2 positively stained both synthetic peptides, whereas ADxNGCT1 only labelled the truncated form. None of the mAbs detected the collagen proteins .
   (**c**) analysis of several synthetic neurogranin peptides that differ in their C-terminus, i.e. intact (Peptide intact C-term) or either truncated at P75 (Peptide trunc P75) or at S76 (Peptide trunc S76), to confirm the specificity of the prototype ELISA towards neurogranin species truncated at P75 at different concentrations .

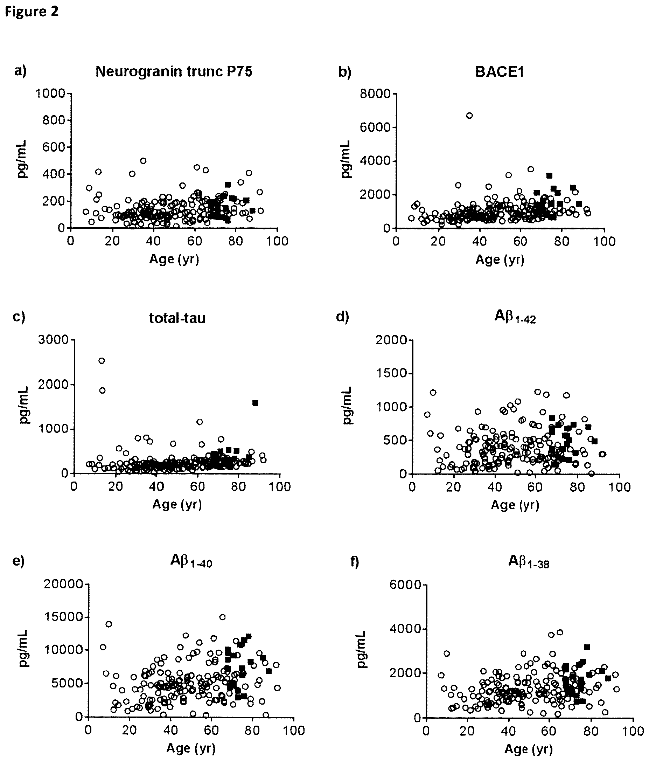

**Fig. 2** shows CSF levels of neurogranin trunc P75 (**a**), BACE1 (**b**), total-tau (**c**), Aβ₁₋₄₂ (**d**), Aβ₁₋₄₀ (**e**) and Aβ₁₋₃₈ (**f**) in 20 cognitively healthy persons (black boxes) and 161 unselected CSF samples within a wide age-range (open circles) plotted against age.

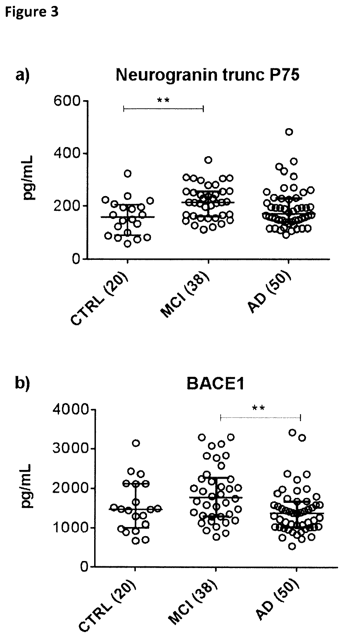

**Fig. 3** shows scatter dot plots demonstrating the concentrations of neurogranin trunc P75 (**a**) or BACE1 (**b**) in CSF in the different diagnostic groups: mild cognitive impairment with high probability for Alzheimer's disease (MCI) (n=38); dementia due to Alzheimer's Disease (AD) (n=50) and cognitively healthy participants (CTRL) (n=20). Presented as a line in each dot plot are the median levels. The bars represent the interquartile range. Statistical significant differences between the groups are presented in the graphs by **= *P*<0.01.

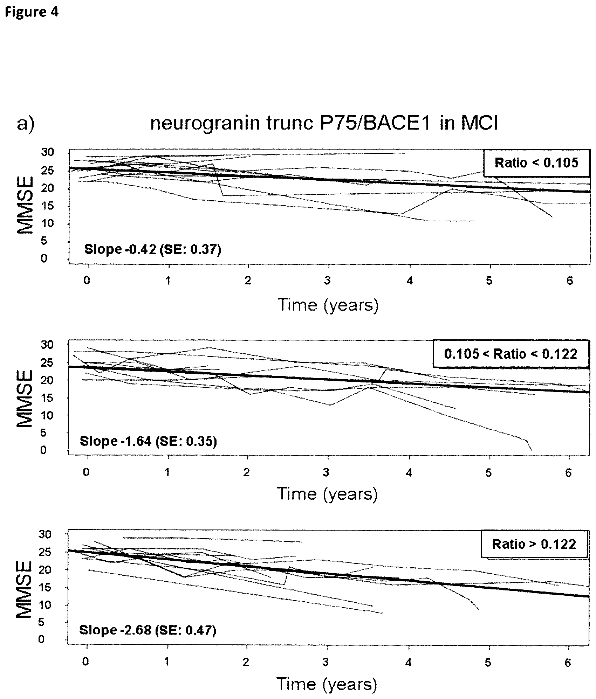

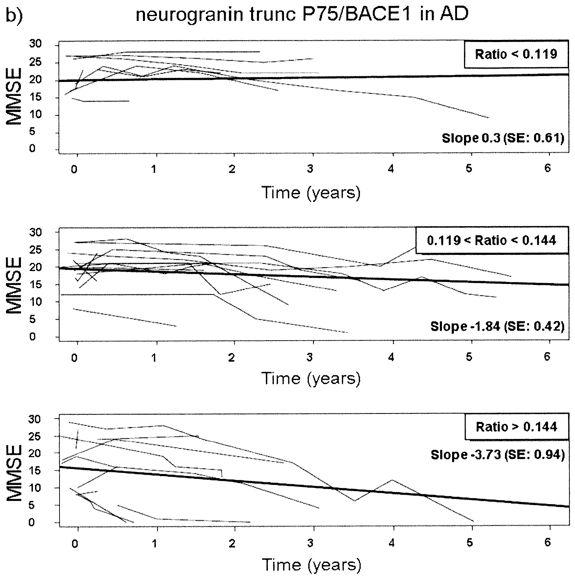

**Fig. 4** shows an illustration of the effect of baseline CSF neurogranin trunc P75/BACE1 levels on the evolution of MMSE over time. Samples were divided into three groups according to the CSF levels of the ratio. The panels show the low (upper panels), medium (mid panels) and high (lower panels) range of levels of CSF neurogranin trunc P75/BACE1. Separate panels were created for both MCI (**a**) and AD patients (**b**). For both the MCI and AD group, the observed MMSE was plotted versus time for each range. The thin lines represent the observed MMSE data for a particular patient, the thick lines symbolise the linear regression line of mean MMSE versus time.

### Examples

### Materials and Methods

### 1.1 Study population

CSF samples were obtained by lumbar puncture (LP) at the L3/L4 or L4/L5 interspace, collected in polypropylene tubes, immediately frozen in liquid nitrogen and stored at -80 °C until analysis. Within 3 months prior to or after LP, neuropsychological examinations, at least consisting of Mini-Mental State Examination (MMSE) scores, were performed for each patient.

Patients with MCI due to AD (referred to as 'MCI' hereafter) (n=38) as well as age-matched patients with dementia due to AD ('AD') (n=50) were included and diagnosed according to the NIA-AA criteria (Albert et al. (2011) The diagnosis of mild cognitive impairment due to Alzheimer's disease: recommendations from the National Institute on Aging-Alzheimer's Association workgroups on diagnostic guidelines for Alzheimer's disease. Alzheimers Dement 7(3), 270-279; McKhann et al. (2011) The diagnosis of dementia due to Alzheimer's disease: recommendations from the National Institute on Aging-Alzheimer's Association workgroups on diagnostic guidelines for Alzheimer's disease. Alzheimers Dement 7(3), 263-269).

To discriminate AD from cognitively normal, CSF biomarkers Aβ₁₋₄₂, T-tau and P-tau181P were already analyzed prior to this study at the BIODEM lab using commercial kits (Innotest β-AMYLOlD(1-42), Innotest hTAU-Ag and Innotest PHOSPHO-TAU(181P); Fujirebio Europe, Ghent, Belgium), whereby the lab's cutoff values were applied [Van der Mussele et al. (2014) Depression in Mild Cognitive Impairment is associated with Progression to Alzheimer's Disease: A Longitudinal Study. J Alzheimers Dis 42(4), 1239-1250]. All MCI patients, of which 2 patients with a pathogenic AD gene mutation, had high probability of AD etiology according to the NIA-AA criteria (see also Supplementary Table 1). Regarding the participants of the age- and gender-matched cognitively healthy control group ('CTRL') (n=20), cognitive deterioration was ruled out by means of a neuropsychological screening. CTRL subjects had no neurological or psychiatric antecedents, central nervous system disorders or inflammatory syndromes.

If available, MMSE total scores from follow-up neuropsychological examinations (at least one year after LP (=baseline) were included in the study. This allowed calculating the rate of cognitive decline based on a linear mixed model adjusted for age and gender.

### 1.2 RESEARCH NEUROGRANIN ELISA FOR CSF

Ninety-six-well microtiter plates were coated with mAb ADxNGCI2 in Phosphate-buffered Saline (PBS), for 1h at 21°C. After washing the wells with PBS containing Tween-20 (PBST), the plates were blocked with blocking reagent for 2 h at 21 °C. The calibrator, a synthetic peptide containing the C-terminus of neurogranin truncated at Pro75 (custom-made by Proteogenix, France), was prepared in sample diluent (SD) (PBS based buffer with blocking reagent and detergents) at an initial concentration of 900 pg/mL and subsequent three-fold dilutions were made. Samples as well as calibrator dilutions were simultaneously incubated during 3 h at 21 °C with the biotinylated mAb ADxNGCT1 in SD. After washing the plates with PBST, horseradish peroxidase-conjugated streptavidin was added. After incubation of 30 min at 21 °C, the plates were washed and a 3,3',5,5'-tetramethylbenzidine solution was added to engage development. After 30 min at 21 °C, the coloring reaction was stopped and plates were read in a BioTek microplate reader, at 450 nm. Levels of neurogranin trunc P75 were calculated via intrapolation (5PL curve fit; log(X)) based on the calibrator curve.

### 1.3 BACE1 ELISA FOR CSF

The BACE1 ELISA used is based on the ELISA method by Barão et al. ((2013) BACE1 levels correlate with phospho-tau levels in human cerebrospinal fluid. Curr Alzheimer Res 10(7), 671-678). This ELISA was further developed by implementing modifications allowing a simultaneous incubation (during 3 h) of 15 µL undiluted CSF sample and the detector antibody. Reagents from EUROIMMUN Medizinische Labordiagnostika AG, Lübeck, Germany, were used (BACE-1 ELISA, product number EQ 6541-9601-L). Also, the inventors performed additional rounds of subcloning of the hybridoma cell line of one of the two involved antibodies to ensure the monoclonal character and production of the antibodies. This resulted in the mAb ADx402 (clone 10B8F1), which is the detector antibody in the ELISA, and which is biotinylated instead of conjugated with peroxidase as in the previous format (described by Barão et al., 2013). The hybridoma cell line of the capture antibody, mAb ADx401 (clone 5G7) underwent no additional rounds of subcloning. The inventors analyzed the BACE1 levels according to the new protocol, where concentrations were calculated via interpolation (5PL curve fit; log (X)) based on a calibrator curve.

### 1.4 ELISA for the CSF biomarkers total-tau, AB1-42, AB1-40 and AB1-38

CSF levels of total-tau, Aβ₁₋₄₂ and Aβ₁₋₄₀ were re-analyzed during this study, using the total-tau ELISA, Beta-Amyloid (1-42) ELISA and Beta-Amyloid (1-40) ELISA kits commercially available from EUROIMMUN Medizinische Labordiagnostika AG, Lübeck, Germany (Beta-Amyloid 1-38, product number EQ 6501-9601 for plasma, EQ 6501-9601-L for CSF; Beta-Amyloid 1-40 ELISA - EQ 6501-9601 for plasma, EQ 6501-9601-L for CSF, Beta-Amyloid 1-42 ELISA, product number EQ 6501-9601 for plasma, EQ 6501-9601-L for CSF).

Also, CSF Aβ₁₋₃₈ was measured using the assay supplied by EUROIMMUN Medizinische Labordiagnostika AG, Lübeck, Germany.

### 1.4 Statistical analysis

GraphPad Prism 6.02 and *R* version 3.1.2 were used for statistical analyses and figures. The strength of the correlation between analytes and/or parameters was expressed by Spearman's correlation coefficient. To compare quantitative variables data between groups, a Kruskal-Wallis test was applied. Linear mixed models were fitted with MMSE as dependent variable, fixed effects included time (continuous variable), CSF marker value and their interaction. The random effects included a random intercept for individual and a random slope for time. The significance of the interaction between the CSF marker value and time entailed whether the level of CSF marker had a significant effect on the MMSE change over time. The significance of this term was tested using an F-test with a Kenward-Roger correction for the number of degrees of freedom. Results were considered significant for *P-*values <0.05.

### Results

### 1.5 GENERATION OF NEUROGRANIN MONOCLONAL ANTIBODIES

The anti-neurogranin mAbs ADxNGCI2 and ADxNGCT1 were generated by immunization of mice with synthetic peptides encompassing the C-terminus of neurogranin. The mAb ADxNGCI2 (isotype IgG2a) was produced at PharmAbs (KU Leuven, Belgium) in Balb/c mice after injections with a KLH-coupled peptide containing an internal sequence of C-terminal neurogranin (R51-D78) and additional boosts with full-length synthetic neurogranin. Both the peptide and full-size neurogranin were synthesized at Proteogenix (France). The mAb ADxNGCT1 (isotype IgG1) was isolated (at BIOTEM, France) from OF1 mice following an immunization with a KLH-conjugated peptide (synthesized at BIOTEM) corresponding to the C-terminus truncated at P75 (G60-P75). These two mAbs ADxNGCI2 and ADxNGCT1 each recognize a different epitope on neurogranin as depicted in **Fig. 1a**. A scan by ELISA with streptavidin coating and biotinylated peptides covering the C-terminal end of neurogranin revealed that the epitope of ADxNGCI2 is located within the sequence R51-A66. The mAb ADxNGCT1 targets the amino acid range from G62, ending at P75. This specificity of both mAbs was also confirmed by gel electrophoresis on synthetic neurogranin, full size as well as truncated at P75. Three abundant types of human collagen were included as well since ADxNGCI2 and ADxNGCT1 target the collagen like domain of neurogranin (**Fig. 1b**).

### 1.6 DESIGN OF AN ELISA TARGETING NEUROGRANIN TRUNCATED AT P75

For measuring neurogranin, the inventors combined ADxNGCI2 as capture antibody and ADxNGCT1 as biotinylated detector antibody, into the research sandwich ELISA specific for neurogranin truncated at P75 (Fig. 1c). This form of neurogranin is more abundant in human CSF than the intact C-terminus [Kvartsberg et al. (2015) Cerebrospinal fluid levels of the synaptic protein neurogranin correlates with cognitive decline in prodromal Alzheimer's disease. Alzheimers Dement 11(10), 1180-1190]. The sensitivity of the new mAbs based assay, was assessed with three CSF samples with low endogenous levels of neurogranin trunc P75, run in 4 replicates and the lowest quantifiable concentration with a coefficient of variation (%CV) of 20 was 26pg/mL (based on the standard curve in duplicate). Three-fold serial dilutions of a synthetic peptide encompassing the C-terminal sequence of neurogranin, ending at P75, were used to generate the standard curve, ranging from 900 to 4pg/mL.

### 1.7 AGE DEPENDENCE OF CSF NEUROGRANIN, CSF BACE1 AND THE CLASSIC CSF AD BIOMARKERS

In preparation of the clinical study, the inventors first verified the neurogranin trunc P75 assay as a proof-of-concept in a large set of CSF samples. Specifically, the inventors analyzed the control group for the clinical study (n=20) in addition to a cohort of diagnostically undefined CSF samples (n=161) (Biomnis, France). The inventors selected the latter group of samples within a wide range of age and with a similar number of male and female subjects. In total, 181 samples (104 female/77 male subjects) were analyzed with a median age of 50.3 (years) (range 7.0 - 92.1). Apart from neurogranin trunc P75, the inventors analyzed the CSF levels of BACE1, tau, Aβ₁₋₄₂, Aβ₁₋₄₀ and Aβ₁₋₃₈. **Table 1** summarizes the concentrations of all analytes. As depicted, only in a very minor number of samples neurogranin trunc P75 levels could not be determined, i.e. 2.8%, which was highly comparable to the other five analytes, i.e. 1.7% for total-tau, Aβ₁₋₄₂ and Aβ₁₋₄₀, 1.1% for Aβ₁₋₃₈, and 0% for BACE1. In addition, all CSF analytes showed an association with age, albeit very weak in case of Aβ₁₋₄₂: neurogranin trunc P75 (p = 0.253; P=0.0007), BACE1 (ρ = 0.412; P<0.0001), tau (p = 0.366; P<0.0001), Aβ₁₋₄₂ (p = 0.147; P=0.050), Aβ₁₋₄₀ (ρ = 0.305; P<0.0001) and Aβ₁₋₃₈ (ρ = 0.326; P<0.0001). **Fig. 2**, which illustrates this relationship between the several CSF analytes and age, also confirms the accurate selection of the age-matched control group for the clinical study. Finally, there was a strong correlation between neurogranin trunc P75, BACE1 and tau on the one hand, and a solid relationship between the three Aβ-species on the other hand (**Table 2**).

### 1.8 CSF LEVELS OF NEUROGRANIN TRUNC P75, BACE1 AND THE CLASSIC CSF AD BIOMARKERS IN MCI AND AD PATIENTS

After establishing the proof-of-concept of the research assay for neurogranin, the inventors investigated the levels of neurogranin trunc P75 and BACE1 in the CSF of MCI (n=38) and AD (n=50) patients versus cognitively normal participants (CTRL) (n=20). In addition, total-tau, Aβ₁₋₄₂, Aβ₁₋₄₀ and Aβ₁₋₃₈ were quantified in the samples, which were tested blinded from clinical diagnosis. **Table 3** sums up the levels of every analyte, combined with the demographic and clinical data of the population.

Compared to the CTRL group, CSF neurogranin trunc P75 was significantly increased in MCI patients (*P*<0.01), but was not significantly different in AD patients (**Table 3**, **Fig. 3**). Likewise, no significant differences were noted between the MCI and AD groups. On the contrary, CSF BACE1 levels were higher in the MCI group compared to the AD patients (*P*<0.01), whereas no significant differences were seen between CTRL and MCI, nor between CTRL and AD. For both proteins, there was a trend of increasing group levels when progressing from cognitively normal to MCI, followed by a decrease in case of progression to AD. These findings are reflected by Receiver Operating Characteristics (ROC) analyses, comparing the CTRL group versus both groups of patients (**Table 4**). CSF neurogranin trunc P75, as single analyte, performed well in discriminating MCI from cognitively healthy with an area under the ROC curve (AUC) of 0.741 (*P*=0.003), but failed to distinguish AD from control (AUC=0.615, ns). Regarding BACE1, no significant discriminating power was noted for CSF BACE1 as single analyte, for MCI (AUC=0.620, ns), nor for AD (AUC=0.554, ns). As expected, the ROC statistics demonstrated strong diagnostic value of the classic CSF biomarkers total-tau and Aβ₁₋₄₂. AUC values for CSF total-tau were 0.842 (*P*<0.0001) in case of MCI and 0.854 (*P*<0.0001) for AD, whereas for CSF Aβ₁₋₄₂, AUC values were 0.765 (*P*=0.001) in case of MCI and 0.849 (*P*<0.0001) for AD. Combining the biomarkers into ratios clearly resulted in higher AUC values as well as increased statistical strength (Table 4). The AUC value for the ratio of CSF neurogranin trunc P75/ Aβ₁₋₄₂ was 0.874 (*P*<0.0001) for discriminating MCI from CTRL and 0.903 (*P*<0.0001) in case of AD versus CTRL, paralleling the largest discriminating performance of the ratio CSF Aβ₁₋₄₂/total-tau (AUC(MCI)=0.871, *P*<0.0001; AUC(AD)=0.923, *P*<0.0001). The combination of both synaptic proteins neurogranin trunc P75 and BACE1 also resulted in an AUC value that was superior to the values of both analytes as single parameter. The AUC value for CSF neurogranin trunc P75/BACE1 was 0.715 (*P*=0.008) regarding MCI patients and 0.806 (*P*<0.0001) for the AD group.

Also, the inventors investigated the relationship between all CSF analytes in the clinical groups, and similar to the proof-of-concept analyses described above, there was a strong correlation between neurogranin trunc P75 and BACE1, in MCI (0.793, *P*<0.0001) as well as AD patients (0.711, *P*<0.0001) (**Table 5**). Moderately strong correlations between these synaptic analytes and total-tau were observed as well, i.e. neurogranin trunc P75 and tau were both correlated in MCI (0.635, *P*<0.0001), and AD (0.617, *P*<0.0001), as were BACE1 and tau in MCI (0.588, *P*<0.001), and AD (0.497, *P*<0.001). Even though neurogranin trunc P75, BACE1 and tau appeared closely associated, subtle differences became apparent when studying their respective associations with the Aβ-species. Neurogranin trunc P75 correlated with both Aβ₁₋₄₀ and Aβ₁₋₃₈ in MCI as well as AD, but in neither case with Aβ₁₋₄₂. On the other hand, tau showed an albeit weak, yet significant correlation with Aβ₁₋₄₂ in MCI (0.383, *P*<0.05), while BACE1 correlated with Aβ₁₋₄₂ in AD (0.408, *P*<0.01). When considering the ratios of CSF Aβ₁₋₄₂/ Aβ₁₋₄₀ and CSF Aβ₁₋₄₂/ Aβ₁₋₃₈ more differences were found (Table 6). In the MCI population, both neurogranin trunc P75 and BACE1 were correlated with both ratios, whereas tau was not.

### 1.9 RELATIONSHIP OF CSF BIOMARKERS WITH MMSE DECLINE

In addition to their diagnostic performance, the inventors evaluated the analytes as possible progression biomarkers by assessing their relationship with cognitive decline. Therefore, the inventors considered the CSF levels of all analytes, as single parameters as well as ratios, in a subset of MCI (n=36) and AD (n=45) patients where MMSE was monitored. Adjusted for age and gender, a linear mixed model analysis, with the longitudinal MMSE measurements as outcome, demonstrated that none of the single analytes at baseline, i.e. neurogranin trunc P75, BACE1 nor the classic CSF AD biomarkers, were associated with the rate of cognitive decline (Table 7). Also the majority of biomarker ratios was not correlated, although, there was one specific ratio of CSF biomarkers showing a consistently significant impact on the longitudinal decline in MMSE in both MCI and AD patients, i.e. CSF neurogranin trunc P75/BACE1 (β=-0.018; *P*<0.05 for MCI; β=-0.051; *P*<0.01 for AD). Statistical significance remained unchanged in case two possible confounders, CSF levels of tau and Aβ₁₋₄₂, were added to the model as fixed effects. To illustrate this correlation between the neurogranin trunc P75/BACE1 ratio and cognitive deterioration, the subset of patients with MMSE at and follow-up after LP was partitioned into three tertiles according to the ratio levels (**Fig 5**). In both the MCI and AD group, individuals (each represented by thin lines) with the highest ratio levels, i.e. in the highest tertile, had the steepest slope or the biggest change in MMSE. This effect was more pronounced in AD patients. There was one additional ratio that was also significantly correlated with cognitive decline, albeit only in AD patients: CSF neurogranin trunc P75/Aβ₁₋₄₂ (β=-3.589; *P*<0.05).

### 2 Results

### 2.1 Generation of Neurogranin monoclonal antibodies

Combining ADxNGCI2 as capture antibody and ADxNGCT1 as biotinylated detector antibody resulted in the research sandwich ELISA specific for neurogranin truncated at P75 (**Fig. 1c**).

### 2.2 Age dependence of CSF Neurogranin, CSF BACE1 and the classic CSF AD biomarkers

In preparation of the clinical study, the inventors first verified the neurogranin trunc P75 assay as a proof-of-concept in a large set of CSF samples. Specifically, the control group for the clinical study (n=20) in addition to a cohort of diagnostically undefined CSF samples (n=161) (Biomnis, France)was analyzed. The latter group of samples within a wide range of age and with a similar number of male and female subjects was selected. In total, 181 samples (104 female/77 male subjects) were analyzed with a median age of 50.3 (years) (range 7.0 - 92.1). Apart from neurogranin trunc P75, we analyzed the CSF levels of BACE1, tau, Aβ₁₋₄₂, Aβ₁₋₄₀ and Aβ₁₋₃₈. **Table 1** summarizes the concentrations of all analytes. As depicted, only in a very minor number of samples neurogranin trunc P75 levels could not be determined, i.e. 2.8 %, which was highly comparable to the other five analytes, i.e. 1.7 % for total-tau, Aβ₁₋₄₂ and Aβ₁₋₄₀, 1.1% for Aβ₁₋₃₈, and 0% for BACE1. In addition, all CSF analytes showed an association with age, albeit very weak in case of Aβ₁₋₄₂: neurogranin trunc P75 (p = 0.253; *P*=0.0007), BACE1 (p = 0.412; *P*<0.0001), tau (p = 0.366; *P*<0.0001), Aβ₁₋₄₂ (ρ = 0.147; *P*=0.050), Aβ₁₋₄₀ (ρ = 0.305; *P*<0.0001) and Aβ₁₋₃₈ (ρ = 0.326; *P*<0.0001). **Fig. 3**, which illustrates this relationship between the several CSF analytes and age, also confirms the accurate selection of the age-matched control group for the clinical study. Finally, there was a strong correlation between neurogranin trunc P75, BACE1 and tau on the one hand, and a solid relationship between the three Aβ species on the other hand (**Table 2**).

### 2.3 CSF levels of Neurogranin trunc 75, BACE1 and the classic AD biomarkers in MCI and AD patients

The inventors investigated the levels of neurogranin trunc P75 and BACE1 in the CSF of MCI (n=38) and AD (n=50) patients versus cognitively normal participants (CTRL) (n=20). In addition, total-tau, Aβ₁₋₄₂, Aβ₁₋₄₀ and Aβ₁₋₃₈ were quantified in the samples, which were tested blinded from clinical diagnosis. **Table 3** sums up the levels of every analyte, combined with the demographic and clinical data of the population.

Compared to the CTRL group, CSF neurogranin trunc P75 was significantly increased in MCI patients (*P*<0.01), but was not significantly different in AD patients (**Table 3**, **Fig. 4**). Likewise, no significant differences were noted between the MCI and AD groups. On the contrary, CSF BACE1 levels were higher in the MCI group compared to the AD patients (P<0.01), whereas no significant differences were seen between CTRL and MCI, nor between CTRL and AD. For both proteins, there was a trend of increasing group levels when progressing from cognitively normal to MCI, followed by a decrease in case of progression to AD. These findings are reflected by Receiver Operating Characteristics (ROC) analyses, comparing the CTRL group versus both groups of patients (**Table 4**). CSF neurogranin trunc P75, as single analyte, performed well in discriminating MCI from cognitively healthy with an area under the ROC curve (AUC) of 0.741 (*P*=0.003), but failed to distinguish AD from control (AUC=0.615, ns). Regarding BACE1, no significant discriminating power was noted for CSF BACE1 as single analyte, for MCI (AUC=0.620, ns), nor for AD (AUC=0.554, ns). As expected, the ROC statistics demonstrated strong diagnostic value of the classic CSF biomarkers total-tau and Aβ₁₋₄₂. AUC values for CSF total-tau were 0.842 (*P*<0.0001) in case of MCI and 0.854 (*P*<0.0001) for AD, whereas for CSF Aβ₁₋₄₂, AUC values were 0.765 (*P*=0.001) in case of MCI and 0.849 (*P*<0.0001) for AD. Combining the biomarkers into ratios clearly resulted in higher AUC values as well as increased statistical strength (Table 4). The AUC value for the ratio of CSF neurogranin trunc P75/ Aβ₁₋₄₂ was 0.874 (*P*<0.0001) for discriminating MCI from CTRL and 0.903 (*P*<0.0001) in case of AD versus CTRL, paralleling the largest discriminating performance of the ratio CSF Aβ₁₋₄₂/total-tau (AUC(MCI)=0.871, *P*<0.0001; AUC(AD)=0.923, *P*<0.0001). The combination of both synaptic proteins neurogranin trunc P75 and BACE1 also resulted in an AUC value that was superior to the values of both analytes as single parameter. The AUC value for CSF neurogranin trunc P75/BACE1 was 0.715 (*P*=0.008) regarding MCI patients and 0.806 (*P*<0.0001) for the AD group.

Also, the inventors investigated the relationship between all CSF analytes in the clinical groups, and similar to the proof-of-concept analyses described above, there was a strong correlation between neurogranin trunc P75 and BACE1, in MCI (0.793, *P*<0.0001) as well as AD patients (0.711, *P*<0.0001) (**Table 5**). Moderately strong correlations between these synaptic analytes and total-tau were observed as well, *i.e.* neurogranin trunc P75 and tau were both correlated in MCI (0.635, *P*<0.0001), and AD (0.617, *P*<0.0001), as were BACE1 and tau in MCI (0.588, *P*<0.001), and AD (0.497, *P*<0.001). Even though neurogranin trunc P75, BACE1 and tau appeared closely associated, subtle differences became apparent when studying their respective associations with the Aβ-species. Neurogranin trunc P75 correlated with both Aβ₁₋₄₀ and Aβ₁₋₃₈ in MCI as well as AD, but in neither case with Aβ₁₋₄₂. On the other hand, tau showed an albeit weak, yet significant correlation with Aβ₁₋₄₂ in MCI (0.383, *P*<0.05), while BACE1 correlated with Aβ₁₋₄₂ in AD (0.408, *P*<0.01). When considering the ratios of CSF Aβ₁₋₄₂/ Aβ₁₋₄₀ and CSF Aβ₁₋₄₂/ Aβ₁₋₃₈ more differences were found (Table 6). In the MCI population, both neurogranin trunc P75 and BACE1 were correlated with both ratios, whereas tau was not.

### 2.4 Relationship of CSF biomarkers with MMSE decline

The inventors considered the CSF levels of all analytes, as single parameters as well as ratios, in a subset of MCI (n=36) and AD (n=41) patients where MMSE was monitored longitudinally post-LP, for at least one year. Adjusted for age and gender, a linear mixed model analysis, with the longitudinal MMSE measurements as outcome, demonstrated that none of the single analytes at baseline, *i.e.* neurogranin trunc P75, BACE1 nor the classic CSF AD biomarkers, were associated with the rate of cognitive decline. However, there was one specific ratio of CSF biomarkers showing a consistently significant impact on the longitudinal decline in MMSE across both the MCI and AD group, **i.e.** CSF neurogranin trunc P75/BACE1 (β=-0.018; *P*<0.05 for MCI; β=-0.051; *P*<0.01 for AD) (**Fig. 5**). Statistical significance remained unchanged in case two possible confounders, CSF levels of tau and Aβ₁₋₄₂, were added to the model as fixed effects. There was one additional ratio that was also significantly correlated with cognitive decline, albeit only in AD patients: CSF neurogranin trunc P75/Aβ₁₋₄₂ (β=-3.589; *P*<0.05).

### Summary

In the sample set of the current study, the inventors have shown that the ratio of CSF Neurogranin trunc P75/BACE1 correlates with ongoing and future cognitive decline in MCI due to AD as well as dementia due to AD, in contrast to neurogranin trunc P75 and BACE1 as single parameter, or in contrast to the current AD CSF biomarkers total-tau, Aβ₄₂ or Aβ₄₀.

**Table 1**

| Overview of all quantified analytes and their correlation with age, in the cohort of unselected CSF samples and the age-matched control group for the clinical study | | | | |
|---|---|---|---|---|
| **Analytes** | **%N** | **Median (p25-p75)** | **Correlation with age (Spearman's p-value)** | ***P*-value of age dependency** |
| CSF neurogranin trunc P75 (pg/mL) | 2.8 | 122 (89-191) | 0.253 | 0.0007 |
| CSF BACE1 (pg/mL) | 0 | 934 (702-1304) | 0.412 | <0.0001 |
| CSF total-tau (pg/mL) | 1.7 | 213 (163-301) | 0.366 | <0.0001 |
| CSF Aβ₁₋₄₂ (pg/mL) | 1.7 | 373 (211-557) | 0.147 | 0.050 |
| CSF Aβ₁₋₄₀ (pg/mL) | 1.7 | 4872 (3177-6772) | 0.305 | <0.0001 |
| CSF Aβ₁₋₃₈ (pg/mL) | 1.1 | 1229 (869-1761) | 0.326 | <0.0001 |

| | | | | |
|---|---|---|---|---|
| %N = percentage of samples not quantifiable (out of 181) | | | | |

**Table 2**

| Spearman's correlation analysis on the CSF biomarkers in the age-matched controls from the clinical study and the cohort of diagnostically unselected samples. Each studied relationship between analytes, represented by Spearman's p-values in the table, was statistically significant (*P*<0.0001). | | | | | | |
|---|---|---|---|---|---|---|
| | **neurogranin trunc P75** | **BACE1** | **total-tau** | **Aβ₁₋₄₂** | **Aβ₁₋₄₀** | **Aβ₁₋₃₈** |
| **neurogranin trunc P75** | | | | | | |
| **BACE1** | 0.746 | | | | | |
| **total-tau** | 0.668 | 0.550 | | | | |
| **Aβ₁₋₄₂** | 0.448 | 0.474 | 0.306 | | | |
| **Aβ₁₋₄₀** | 0.595 | 0.625 | 0.488 | 0.888 | | |
| **Aβ₁₋₃₈** | 0.658 | 0.688 | 0.519 | 0.806 | 0.948 | |

**Table 3**

| Summary of the demographic, analytes are summarized as me between the age-matched cont Kruskal-Wallis test. clinical and biomarker data dian values with 25th and rol group (CTRL) and the of the clinical groups, 75th quartiles. Median MCI and AD groups were where data on the differences tested using a | | | |
|---|---|---|---|
| | **CTRL** | **MCI** | **AD** |
| **Demographic** | | | |
| Gender, N (F/M) | 20 (10/10) | 38 (23/15) | 50 (27/23) |
| Age at LP (yr) | 74 (69-76) | 73 (69-79) | 75 (68-78) |

| **Clinical** | | | |
|---|---|---|---|
| MMSE at LP (/30) | 27 (24-30) | 25 (23-27) | 18 (12-23) |

| **Biochemical** | | | |
|---|---|---|---|
| CSF neurogranin trunc P75 (pg/mL) | 159 (92-205) | 214 (161-256)^{∗∗} | 172 (141-230) |
| CSF BACE1 (pg/mL) | 1472 (1012-2121) | 1777 (1291-2276) | 1378 (1031-1679) |
| CSF total-tau (pg/mL) | 349 (242-434) | 567 (446-706)^{∗∗∗∗} | 535 (454-711)^{∗∗∗∗} |
| CSF Aβ₁₋₄₂ (pg/mL) | 503 (267-687) | 283 (219-350)^{∗} | 208 (166-287)^{∗∗∗∗} |
| CSF Aβ₁₋₄₀ (pg/mL) | 7352 (4884-9514) | 7685 (6604-9881) | 5662 (4307-7413) |
| CSF Aβ₁₋₃₈ (pg/mL) | 1728 (1206-2169) | 1802 (1539-2385) | 1316 (1075-1659) |
| CSF Aβ₁₋₄₂/total-tau | 1.539 (0.911-1.916) | 0.502 (0.380-0.622)^{∗∗∗} | 0.354 (0.290-0.470)^{∗∗∗∗} |
| CSF Aβ₁₋₄₂/Aβ₁₋₄₀ | 0.070 (0.052-0.080) | 0.035 (0.028-0.040)^{∗∗∗∗} | 0.038 (0.029-0.045)^{∗∗∗∗} |
| CSF Aβ₁₋₄₂/Aβ₁₋₃₈ | 0.286 (0.225-0.364) | 0.154 (0.111-0.179)^{∗∗∗∗} | 0.162 (0.115-0.191)^{∗∗∗∗} |
| CSF neurogranin trunc P75/BACE1 | 0.095 (0.088-0.111) | 0.120 (0.101-0.142)^{∗} | 0.136 (0.114-0.157)^{∗∗∗} |
| CSF neurogranin trunc P75/Aβ₁₋₄₂ | 0.297 (0.228-0.492) | 0.767 (0.560-1.026)^{∗∗∗∗} | 0.843 (0,626-1.135)^{∗∗∗∗} |

| | | | |
|---|---|---|---|
| *AD* = Alzheimer's disease; *CTRL* = cognitively healthy; *F* = female; *LP* = lumbar puncture; *M* = Male; *MCI* = Mild Cognitive Impairment with high probability for AD; *MMSE* = Mini-Mental State Examination; *N* = number of samples; *yr* = years The statistical significance (versus CTRL) is represented by ^{∗} *P*<0.05; ^{∗∗} *P*<0.01; ^{∗∗∗} *P*<0.001; ^{∗∗∗∗} *P*<0.0001. | | | |

**Table 4**

| ROC-statistics on the CSF biomarkers as single analytes or as ratio, where the set of data of the CTRL group was aligned with the MCI or AD group. The P-values <0.05 are emphasized in bolt font. | | | | |
|---|---|---|---|---|
| | **MCI vs CTRL (n=38)** | | **AD vs CTRL (n=50)** | |
| **Single analyte** | **AUC** | ***P*-value** | **AUC** | **P-value** |
| neurogranin trunc P75 | 0.741 | **0.003** | 0.615 | 0.137 |
| BACE1 | 0.620 | 0.137 | 0.554 | 0.487 |
| total-tau | 0.842 | **<0.0001** | 0.854 | **<0.0001** |
| Aβ₁₋₄₂ | 0.765 | **0.001** | 0.849 | **<0.0001** |
| Aβ₁₋₄₀ | 0.559 | 0.467 | 0.660 | **0.038** |
| Aβ₁₋₃₈ | 0.622 | 0.130 | 0.645 | 0.059 |
| **Ratio of analytes** | **AUC** | ***P*-value** | **AUC** | ***P*-value** |
| Aβ₁₋₄₂/Aβ₁₋₄₀ | 0.904 | **<0.0001** | 0.887 | **<0.0001** |
| Aβ₁₋₄₂/Aβ₁₋₃₈ | 0.894 | **<0.0001** | 0.876 | **<0.0001** |
| Aβ₁₋₄₂/total-tau | 0.871 | **<0.0001** | 0.923 | **<0.0001** |
| neurogranin trunc P75/Aβ₁₋₄₂ | 0.874 | **<0.0001** | 0.903 | **<0.0001** |
| neurogranin trunc P75/BACE1 | 0.715 | **0.008** | 0.806 | **<0.0001** |

| | | | | |
|---|---|---|---|---|
| *AD* = Alzheimer's disease; *AUC* = Area Under the ROC; *CTRL* = cognitively healthy; *F* = female; *M* = Male; *MCI* = Mild Cognitive Impairment with high probability for AD; *MMSE* = Mini-Mental State Examination; *N* = number of samples; *ROC* = Receiver Operating Curve; *yr =* years | | | | |

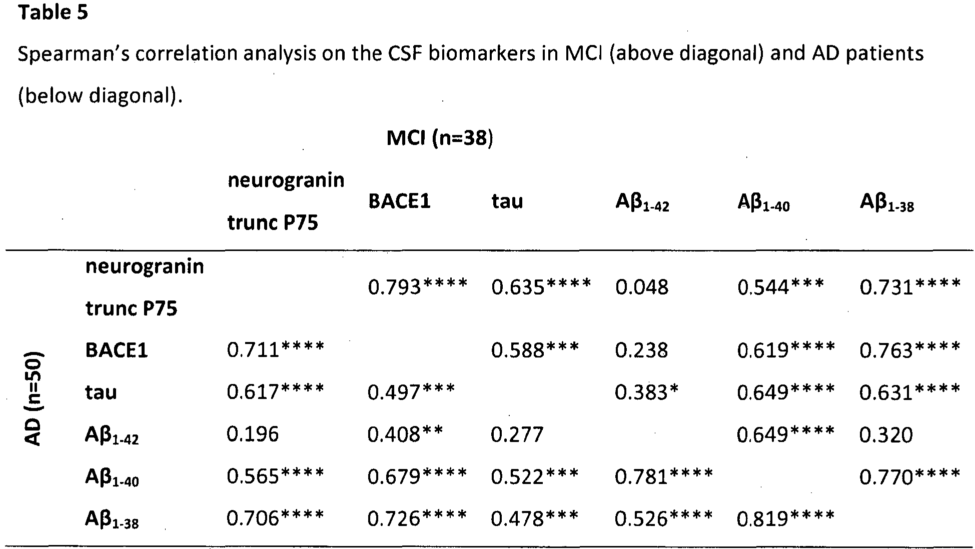

*AD* = Alzheimer's disease; *MCI* = Mild Cognitive Impairment with high probability for AD.

The statistical significance is represented by ^{∗} *P*<0.05; ^{∗∗} *P*<0.01; ^{∗∗∗} *P*<0.001; ^{∗∗∗∗} *P*<0.0001.

**Table 6**

| Spearman's correlation analysis on CSF neurogranin trunc P75, BACE1 and total-tau with the ratios CSF Aβ₁₋₄₂/Aβ₁₋₄₀ and CSF Aβ₁₋₄₂/Aβ₁₋₃₈ in CTRL as well as MCI and AD. | | | | | | |
|---|---|---|---|---|---|---|
| | | **Aβ₁₋₄₂/ Aβ₁₋₄₀** | | | **Aβ₁₋₄₂/ Aβ₁₋₃₈** | |
| | **CTRL** | **MCI** | | **CTRL** | **MCI** | **AD** |
| | | | **AD (n=50)** | | | |
| | **(n=20)** | **(n=38)** | | **(n=20)** | **(n=38)** | **(n=50)** |
| **neurogranin trunc** | | - | - - | -0.430 | | -0.365^{∗∗} |
| | -0.371 | | | | | |
| **P75** | | 0.608^{∗∗∗∗} | 0.651^{∗∗∗∗} | | 0.662^{∗∗∗∗} | |
| **BACE1** | -0.063 | -0.485^{∗∗} | -0.497^{∗∗∗} | -0.140 | -0.551^{∗∗∗} | -0.167 |
| **total-tau** | 0.092 | -0.275 | -0.465^{∗∗∗} | -0.012 | -0.293 | -0.080 |

| | | | | | | |
|---|---|---|---|---|---|---|
| *AD* = Alzheimer's disease; *CTRL* = cognitively healthy; *MCI* = Mild Cognitive Impairment with high probability for AD. The corresponding statistical significance is represented by ^{∗∗} *P*<0.01;^{∗∗∗} *P*<0.001; ^{∗∗∗∗} *P*<0.0001. | | | | | | |

**Table 7**

| Statistical significance of the association between cognitive decline and the single analytes and ratios, based on linear mixed model analysis (MCI n=36; AD n=45), adjusted for age and gender. The P-values <0.05 are underlined and emphasized in bolt font. | | |
|---|---|---|
| | **MCI (n=36)** | **AD (n=45)** |
| **Single analyte** | ***P*-value** | ***P*-value** |
| total-tau | 0.684 | 0.245 |
| Aβ₁₋₄₀ | 0.534 | 0.927 |
| Aβ₁₋₄₂ | 0.192 | 0.490 |
| Aβ₁₋₃₈ | 0.888 | 0.493 |
| BACE1 | 0.524 | 0.996 |
| Neurogranin trunc P75 | 0.766 | 0.203 |

| **Ratio of analytes** | ***P*-value** | ***P*-value** |
|---|---|---|
| Aβ_{1-42/}Aβ₁₋₄₀ | 0.434 | 0.605 |
| Aβ₁₋₄₂/total-tau | 0.122 | 0.092 |
| Neurogranin trunc P75/Aβ₁₋₄₂ | 0.286 | **0.023** |
| Neurogranin trunc P75/total-tau | 0.999 | 0.615 |
| BACE1/Aβ₁₋₄₂ | 0.888 | 0.458 |
| BACE1/total-tau | 0.099 | 0.098 |
| Neurogranin trunc P75/BACE1 | **0.036** | **0.005** |

### References:

Albert et al. (2011) The diagnosis of mild cognitive impairment due to Alzheimer's disease: recommendations from the National Institute on Aging-Alzheimer's Association workgroups on diagnostic guidelines for Alzheimer's disease. Alzheimers Dement 7(3), 270-279.
McKhann et al. (2011) The diagnosis of dementia due to Alzheimer's disease: recommendations from the National Institute on Aging-Alzheimer's Association workgroups on diagnostic guidelines for Alzheimer's disease. Alzheimers Dement 7(3), 263-269.
Jack et al. (2013) Tracking pathophysiological processes in Alzheimer's disease: an updated hypothetical model of dynamic biomarkers. Lancet Neurol 12(2), 207-216.
Duits et al. (2014) The cerebrospinal fluid "Alzheimer profile": easily said, but what does it mean? Alzheimers Dement 10(6), 713-723.
Lewczuk et al. (2015) Amyloid-β 42/40 cerebrospinal fluid concentration ratio in the diagnostics of Alzheimer's disease: validation of two novel assays. J Alzheimers Dis 43(1), 183-191.
Sauvée et al. (2014) Additional use of Aβ42/Aβ40 ratio with cerebrospinal fluid biomarkers P-tau and Aβ42 increases the level of evidence of Alzheimer's disease pathophysiological process in routine practice. J Alzheimers Dis 41(2), 377-386.
Reddy et al. (2005) Differential loss of synaptic proteins in Alzheimer's disease: implications for synaptic dysfunction. J Alzheimers Dis 7(2), 103-117; discussion 173-180.
Selkoe DJ (2002) Alzheimer's disease is a synaptic failure. Science 298(5594), 789-791.
Terry RD, Masliah E, Salmon DP, Butters N, DeTeresa R, Hill R, Hansen LA, Katzman R (1991) Physical basis of cognitive alterations in Alzheimer's disease: synapse loss is the major correlate of cognitive impairment. Ann Neurol 30(4), 572-580.
Díez-Guerra (2010) Neurogranin, a link between calcium/calmodulin and protein kinase C signaling in synaptic plasticity. IUBMB Life 62(8), 597-606.
Represa et al. (1990) Neurogranin: immunocytochemical localization of a brain-specific protein kinase C substrate. J Neurosci 10(12), 3782-3792.
Watson et al. (1992) Localization of the protein kinase C phosphorylation/calmodulin-binding substrate RC3 in dendritic spines of neostriatal neurons. Proc Natl Acad Sci U S A 89(18), 8581-8585. Zhong et al. (2009) Neurogranin enhances synaptic strength through its interaction with calmodulin. EMBO J 28(19), 3027-3039.
Baudier et al. (1991) Purification and characterization of a brain-specific protein kinase C substrate, neurogranin (p17). Identification of a consensus amino acid sequence between neurogranin and neuromodulin (GAP43) that corresponds to the protein kinase C phosphorylation site and the calmodulin-binding domain. J Biol Chem 266(1), 229-237.
Sjöstedt et al.(2015) Defining the Human Brain Proteome Using Transcriptomics and Antibody-Based Profiling with a Focus on the Cerebral Cortex. PLoS One 10(6), e0130028. doi:10.1371/journal.pone.0130028.
Kvartsberg et al. (2015) Cerebrospinal fluid levels of the synaptic protein neurogranin correlates with cognitive decline in prodromal Alzheimer's disease. Alzheimers Dement 11(10), 1180-1190.
De Vos et al. (2015) C-terminal neurogranin is increased in cerebrospinal fluid but unchanged in plasma in Alzheimer's disease. Alzheimers Dement 11(12), 1461-1469.
Janelidze et al. (2015) Cerebrospinal fluid neurogranin and YKL-40 as biomarkers of Alzheimer's disease. Ann Clin Transl Neurol 3(1), 12-20. doi: 10.1002/acn3.266. eCollection 2016 Jan.
Kester et al. (2015) Neurogranin as a Cerebrospinal Fluid Biomarker for Synaptic Loss in Symptomatic Alzheimer Disease. JAMA Neurol 72(11), 1275-1280. doi:10.1001/jamaneurol.2015.1867.
Thorsell et al. (2010) Neurogranin in cerebrospinal fluid as a marker of synaptic degeneration in Alzheimer's disease. Brain Res 1362, 13-22. doi:10.1016/j.brainres.2010.09.073.
Portelius et al. (2015) Cerebrospinal fluid neurogranin: relation to cognition and neurodegeneration in Alzheimer's disease. Brain 138(Pt 11), 3373-3385. doi:10.1093/brain/awv267.
Kvartsberg et al. (2015) Characterization of the postsynaptic protein neurogranin in paired cerebrospinal fluid and plasma samples from Alzheimer's disease patients and healthy controls. Alzheimers Res Ther 7(1), 40. doi:10.1186/s13195-015-0124-3.
Kamenetz et al. (2003) APP processing and synaptic function. Neuron 37(6), 925-937.
Kandalepas et al. (2013) The Alzheimer's β-secretase BACE1 localizes to normal presynaptic terminals and to dystrophic presynaptic terminals surrounding amyloid plaques. Acta Neuropathol 126(3), 329-352.
Vassar et al. (1999) Beta-secretase cleavage of Alzheimer's amyloid precursor protein by the transmembrane aspartic protease BACE. Science 286(5440), 735-741.
Ahmed et al. (2010) BACE1 and BACE2 enzymatic activities in Alzheimer's disease. J Neurochem 112(4), 1045-1053.
Fukumoto et al. (2002) Beta-secretase protein and activity are increased in the neocortex in Alzheimer disease. Arch Neurol 59(9), 1381-1389.
Barao et al. (2013) BACE1 levels correlate with phospho-tau levels in human cerebrospinal fluid. Curr Alzheimer Res 10(7), 671-678.
Pera et al. (2013) Distinct patterns of APP processing in the CNS in autosomal-dominant and sporadic Alzheimer disease. Acta Neuropathol 125(2), 201-213.
Rosen et al. (2012) Cerebrospinal fluid profiles of amyloid β-related biomarkers in Alzheimer's disease. Neuromolecular Med 14(1), 65-73.
Savage et al. (2015) Soluble BACE-1 Activity and sAβPPβ Concentrations in Alzheimer's Disease and Age-Matched Healthy Control Cerebrospinal Fluid from the Alzheimer's Disease Neuroimaging Initiative-1 Baseline Cohort. J Alzheimers Dis 46(2), 431-440.
Zetterberg et al. (2008) Elevated cerebrospinal fluid BACE1 activity in incipient Alzheimer disease. Arch Neurol 65(8), 1102-1107.
Zhong et al. (2007) Levels of beta-secretase (BACE1) in cerebrospinal fluid as a predictor of risk in mild cognitive impairment. Arch Gen Psychiatry 64(6), 718-726.
Mulder et al. (2010) BACE1 activity in cerebrospinal fluid and its relation to markers of AD pathology. J Alzheimers Dis 20(1), 253-260.
Van der Mussele et al. (2014) Depression in Mild Cognitive Impairment is associated with Progression to Alzheimer's Disease: A Longitudinal Study. J Alzheimers Dis 42(4), 1239-1250.
Montine et al. (2012) National Institute on Aging-Alzheimer's Association guidelines for the neuropathologic assessment of Alzheimer's disease: a practical approach. Acta Neuropathol 123(1), 1-11.
Braak & Braak E (1991) Neuropathological stageing of Alzheimer-related changes. Acta Neuropathol 82(4), 239-259.
Thal DR, Rüb U, Orantes M, Braak H (2002) Phases of A beta-deposition in the human brain and its relevance for the development of AD. Neurology 58(12), 1791-1800.
Wayne PA (2000) Clinical and Laboratory Standards Institute. How to define and determine reference intervals in the clinical laboratory: approved guideline. CLSI document C28-A2.
Teunissen et al. (2013) Consensus definitions and application guidelines for control groups in cerebrospinal fluid biomarker studies in multiple sclerosis. Mult Scler 19(13), 1802-1809.
Slaets et al (2013) Cerebrospinal fluid Aβ1-40 improves differential dementia diagnosis in patients with intermediate P-tau181P levels. J Alzheimers Dis 36(4), 759-767.
Bateman et al. (2012) Clinical and biomarker changes in dominantly inherited Alzheimer's disease. N Engl J Med 367(9), 795-804.
Palmqvist et al. (2015) Detailed comparison of amyloid PET and CSF biomarkers for identifying early Alzheimer disease. Neurology 85(14), 1240-1249.
Brinkmalm et al. (2014) SNAP-25 is a promising novel cerebrospinal fluid biomarker for synapse degeneration in Alzheimer's disease. Mol Neurodegener 9:53. doi: 10.1186/1750-1326-9-53. Coleman & Yao(2003) Synaptic slaughter in Alzheimer's disease. Neurobiol Aging 24(8), 1023-1027. Blennow et al. (1996) Synaptic pathology in Alzheimer's disease: relation to severity of dementia, but not to senile plaques, neurofibrillary tangles, or the ApoE4 allele. J Neural Transm (Vienna) 103(5), 603-618.
Pannee et al. (2016) Round robin test on quantification of amyloid-β 1-42 in cerebrospinal fluid by mass spectrometry. Alzheimers Dement 12(1), 55-59.
Bjerke et al. (2015) Assessing the commutability of reference material formats for the harmonization of amyloid beta measurements. Clin Chem Lab Med doi: 10.1515/cclm-2015-0733.
Zhabotinsky et al. (2006) Role of the Neurogranin Concentrated in Spines in the Induction of Long-term Potentiation. J NeuroSci 26(28), 7337-47.
Alvarez-Bolado et al. (1996) Neurogranin in the development of the rat telencephalon. Neuroscience 73(2), 565-580.
Fredolini et al. (2016) Immunocapture strategies in translational proteomics, Expt. Rev. Proteomics 13(1), 83-98Ladenson et al. (2007) Markers for brain damage, EP 1 774 023
Zhou et al. (2011) Inhibition of beta-secretase in vivo via antibody binding to unique loops (D and F) of BACE1. J Biol Chem 286(10),8677-8687.
Rosseels et al. (2015) Tau monoclonal antibody generation based on humanized yeast models: impact on Tau oligomerization and diagnostics. J Biol Chem 290(7),4059-4074.

## Claims

1. A diagnostically useful carrier comprising a means for capturing Neurogranin and a means for capturing BACE1, wherein the means for capturing BACE1 is an antibody or aptamer and the means for capturing Neurogranin is an antibody or aptamer binding to a C-terminally truncated fragment of Neurogranin that comprises at least SEQ ID NO 4, but lacks the three C-terminal residues of full-length Neurogranin, namely S76, G77 and D78.

2. The diagnostically useful carrier according to claim 1 comprising a means for capturing one or more additional biomarkers, wherein the means for capturing the one or more additional biomarkers is an antibody or aptamer.

3. A kit comprising the diagnostically useful carrier according to claim 1 or 2.

4. The kit or carrier according to any of claims 1 to 3, wherein the means for specifically capturing Neurogranin and the means for capturing BACE1 are spatially separated.

5. The kit or carrier according to claim 4, wherein the diagnostically useful carrier is selected from the group comprising a bead, a test strip, a microtiter plate, a blot, a glass surface, a slide, a biochip, a membrane, a microarray, an electrophoresis gel and a submicrometer sized particle.

6. A method comprising the steps
(a1) Contacting a sample from a subject with a means for capturing Neurogranin,
(b1) Isolating the means for capturing Neurogranin from the sample and
(a2) Contacting a sample from a subject with a means for capturing BACE1 and
(b2) Isolating the means for capturing BACE1 from the sample,
wherein the means for capturing BACE1 is an antibody or aptamer and and the means for capturing Neurogranin is an antibody or aptamer binding to a C-terminally truncated fragment of Neurogranin that comprises at least SEQ ID NO 4, but lacks the three C-terminal residues of full-length Neurogranin, namely S76, G77 and D78.

7. The method according to claim 6, further comprising the steps
(c1) Contacting the means for capturing Neurogranin with a detectable ligand binding to Neurogranin and
(c2) Contacting the means for capturing BACE1 with a detectable ligand binding to BACE1.

8. The method according to any of claims 6 to 7, further comprising the steps
(d1) Detecting any Neurogranin captured and
(d2) Detecting any BACE1 captured.

9. The method according to any of claims 6 to 8, further comprising the steps
(a3) Contacting a sample from a subject with a means for capturing one or more additional biomarkers,
(b3) Isolating the means for capturing the one or more additional biomarkers from the sample, and
wherein the means for capturing the one or more additional biomarkers is an antibody or aptamer.

10. The carrier, kit or method according to any of claims 2 to 5 and 9, wherein the one or more additional biomarkers are selected from the group comprising Abeta42 and Abeta40, p-tau, t-tau and neurofilament protein.

11. The carrier, kit or method according to one of claims 1 to 10,
wherein the carrier, kit or method is adapted for detecting Neurogranin and BACE1 using a method from the group comprising immunodiffusion techniques; immunoelectrophoretic techniques; light scattering immunoassays; agglutination techniques; labeled immunoassays; mass spectrometry; and Surface Plasmon Resonance.

12. The carrier, kit or method according to claim 11, wherein the carrier, kit or method is adapted for detecting Neurogranin, BACE1 based on a sandwich ELISA.

13. A use of the diagnostically useful carrier or kit according to any of claims 1 to 5 for diagnosing a neurological or neurodegenerative disease.

14. The use according to claim 13, wherein the diagnosis aims to predict the rate of future cognitive decline.

15. The use according to claim 13, wherein the diagnosis aims to distinguish a neurodegenerative disease and depression with or without cognitive impairment.

## Patentansprüche

1. Diagnostisch nützlicher Träger umfassend ein Mittel zum Einfangen von Neurogranin und ein Mittel zum Einfangen von BACE1, wobei das Mittel zum Einfangen von BACE1 ein Antikörper oder Aptamer und das Mittel zum Einfangen von Neurogranin ein Antikörper oder Aptamer ist, der oder das an ein C-terminal verkürztes Neurogranin-Fragment bindet, welches wenigstens SEQ ID NO 4 umfasst, dem aber die drei C-terminalen Reste des Volllängen-Neurogranins fehlen, nämlich S76, G77 and D78.

2. Diagnostisch nützlicher Träger nach Anspruch 1 umfassend ein Mittel zum Einfangen von einem oder mehr als einem zusätzlichen Biomarker, wobei das Mittel zum Einfangen des einen oder der mehr als einen zusätzlichen Biomarker ein Antikörper oder Aptamer ist.

3. Kit umfassend den diagnostisch nützlichen Träger nach Anspruch 1 oder 2.

4. Kit oder Träger nach einem der Ansprüche 1 bis 3, wobei das Mittel zum spezifischen Einfangen von Neurogranin und das Mittel zum Einfangen von BACE1 räumlich getrennt sind.

5. Kit oder Träger nach Anspruch 4, wobei der diagnostisch nützliche Träger aus der Gruppe umfassend einen Bead, einen Teststreifen, eine Mikrotiterplatte, einen Blot, eine Glasoberfläche, einen Objektträger, einen Biochip, eine Membran, einen Mikroarray, ein Elektrophoresegel und einen Partikel mit einer Größe im Submikrometerbereich ausgewählt ist.

6. Verfahren umfassend die Schritte
(a1) Kontaktieren einer Probe von einem Subjekt mit einem Mittel zum Einfangen von Neurogranin,
(b1) Isolieren des Mittels zum Einfangen von Neurogranin aus der Probe und
(a2) Kontaktieren einer Probe von einem Subjekt mit einem Mittel zum Einfangen von BACE1 und
(b2) Isolieren des Mittels zum Einfangen von BACE1 aus der Probe,
wobei das Mittel zum Einfangen von BACE1 ein Antikörper oder Aptamer und das Mittel zum Einfangen von Neurogranin ein Antikörper oder Aptamer ist, der oder das an ein C-terminal verkürztes Neurogranin-Fragment bindet, welches wenigstens SEQ ID NO 4 umfasst, dem aber die letzten drei C-terminalen Reste des Volllängen-Neurogranins fehlen, nämlich S76, G77 and D78.

7. Verfahren nach Anspruch 6, weiter umfassend die Schritte
(c1) Kontaktieren des Mittels zum Einfangen von Neurogranin mit einem detektierbaren, an Neurogranin bindenden Liganden und
(c2) Kontaktieren des Mittels zum Einfangen von BACE1 mit einem detektierbaren, an BACE1 bindenden Liganden.

8. Verfahren nach einem der Ansprüche 6 bis 7, weiter umfassend die Schritte
(d1) Nachweisen von eingefangenem Neurogranin und
(d2) Nachweisen von eingefangenem BACE1.

9. Verfahren nach einem der Ansprüche 6 bis 8, weiter umfassend die Schritte
(a3) Kontaktieren einer Probe von einem Subjekt mit einem Mittel zum Einfangen von einem oder mehr als einem zusätzlichen Biomarker,
(b3) Isolieren des Mittels zum Einfangen des einen oder der mehr als einen zusätzlichen Biomarker von der Probe, und
wobei das Mittel zum Einfangen des einen oder der mehr als einen zusätzlichen Biomarker ein Antikörper oder Aptamer ist.

10. Träger, Kit oder Verfahren nach einem der Ansprüche 2 bis 5 und 9, wobei der eine oder mehr als eine zusätzliche Biomarker aus der Gruppe umfassend Abeta42 und Abeta40, p-tau, t-tau und Neurofilament Protein ausgewählt sind.

11. Träger, Kit oder Verfahren nach einem der Ansprüche 1 bis 10,
wobei der Träger, das Kit oder das Verfahren zum Nachweisen von Neurogranin und BACE1 unter Verwendung eines Verfahrens aus der Gruppe umfassend Techniken der Immundiffusion, immunelektrophoretische Techniken, Lichtstreuungsimmunassays, Agglutinations-Techniken, markierte Immunassays, Massenspektrometrie und Oberflächenplasmonenresonanz eingerichtet ist.

12. Träger, Kit oder Verfahren nach Anspruch 11, wobei der Träger, das Kit oder das Verfahren für das Nachweisen von Neurogranin, BACE1 basierend auf einem Sandwich-ELISA eingerichtet ist.

13. Verwendung des diagnostisch nützlichen Trägers oder des Kits nach einem der Ansprüche 1 bis 5 zum Diagnostizieren einer neurologischen oder neurodegenerativen Erkrankung.

14. Verwendung nach Anspruch 13, wobei die Diagnose darauf abzielt, die Geschwindigkeit des zukünftigen kognitiven Abbaus vorherzusagen.

15. Verwendung nach Anspruch 13, wobei die Diagnose darauf abzielt, eine neurodegenerative Erkrankung von einer Depression mit oder ohne kognitiver Beeinträchtigung zu unterscheiden.

## Revendications

1. Porteur utile en diagnostic comprenant un moyen destiné à capturer la neurogranine et un moyen destiné à capturer la BACE1, dans lequel le moyen destiné à capturer la BACE1 est un anticorps ou un aptamère et le moyen destiné à capturer la neurogranine est un anticorps ou un aptamère se liant à un fragment tronqué à l'extrémité C-terminale de la neurogranine, qui comprend au moins la SEQ ID n° : 4 mais qui est dépourvu des trois résidus C-terminaux de la neurogranine de longueur totale, à savoir les S76, G77 et D78.

2. Porteur utile en diagnostic selon la revendication 1, comprenant un moyen destiné à capturer un ou plusieurs biomarqueur(s) supplémentaire(s), dans lequel le moyen destiné à capturer le ou les plusieurs biomarqueur(s) supplémentaire(s) est un anticorps ou un aptamère.

3. Trousse comprenant le porteur utile en diagnostic selon les revendications 1 ou 2.

4. Trousse ou porteur selon l'une quelconque des revendications 1 à 3, dans laquelle/lequel le moyen destiné à capturer spécifiquement la neurogranine et le moyen destiné à capturer la BACE1 sont séparés dans l'espace.

5. Trousse ou porteur selon la revendication 4, le porteur utile en diagnostic étant choisi dans le groupe comprenant une bille, une bandelette de test, une plaque de microtitration, un transfert, une surface en verre, une lame, une biopuce, une membrane, un micro-arrangement, un gel d'électrophorèse et une particule à taille submicronique.

6. Procédé comprenant les étapes
(a1) de mise en contact d'un échantillon provenant d'un sujet avec un moyen destiné à capturer la neurogranine,
(b1) d'isolement, à partir de l'échantillon, du moyen destiné à capturer la neurogranine et
(a2) de mise en contact d'un échantillon provenant d'un sujet avec un moyen destiné à capturer la BACE1 et
(b2) d'isolement, à partir de l'échantillon, du moyen destiné à capturer la BACE1,
dans lequel le moyen destiné à capturer la BACE1 est un anticorps ou un aptamère et le moyen destiné à capturer la neurogranine est un anticorps ou un aptamère se liant à un fragment tronqué à l'extrémité C-terminale de la neurogranine, qui comprend au moins la SEQ ID n° : 4 mais qui est dépourvu des trois résidus C-terminaux de la neurogranine de longueur totale, à savoir les S76, G77 et D78.

7. Procédé selon la revendication 6, comprenant en outre les étapes
(c1) de mise en contact du moyen destiné à capturer la neurogranine avec un ligand détectable se liant à la neurogranine et
(c2) de mise en contact du moyen destiné à capturer la BACE1 avec un ligand détectable se liant à la BACE1.

8. Procédé selon l'une quelconque des revendications 6 à 7, comprenant en outre les étapes
(d1) de détection d'une quelconque neurogranine capturée et
(d2) de détection d'une quelconque BACE1 capturée.

9. Procédé selon l'une quelconque des revendications 6 à 8, comprenant en outre les étapes
(a3) de mise en contact d'un échantillon provenant d'un sujet avec un moyen destiné à capturer un ou plusieurs biomarqueur(s) supplémentaire(s),
(b3) d'isolement, à partir de l'échantillon, du moyen destiné à capturer le ou les plusieurs biomarqueur(s) supplémentaire(s) et
dans lequel le moyen destiné à capturer le ou les plusieurs biomarqueur(s) supplémentaire(s) est un anticorps ou un aptamère.

10. Porteur, trousse ou procédé selon l'une quelconque des revendications 2 à 5 et 9, dans lequel/laquelle le ou les biomarqueur(s) supplémentaire(s) est/sont choisi(s) dans le groupe comprenant les Abêta42 et Abêta40, p-tau, t-tau et la protéine des neurofilaments.

11. Porteur, trousse ou procédé selon l'une des revendications 1 à 10,
le porteur, la trousse ou le procédé étant adapté(e) à une détection de neurogranine et de BACE1 en utilisant un procédé choisi dans le groupe comprenant des techniques d'immunodiffusion ; des techniques immuno-électrophorétiques ; des immuno-dosages à diffusion de lumière ; des techniques d'agglutination ; des immuno-dosages marqués ; la spectrométrie de masse ; et la résonance plasmonique de surface.

12. Porteur, trousse ou procédé selon la revendication 11, le porteur, la trousse ou le procédé étant adapté(e) à une détection de neurogranine, de BACE1 sur la base d'un ELISA "sandwich.

13. Utilisation du porteur utile en diagnostic ou de la trousse, selon l'une quelconque des revendications 1 à 5, pour diagnostiquer une maladie neurologique ou neurodégénérative.

14. Utilisation selon la revendication 13, dans laquelle le diagnostic a pour but de prédire le degré de déclin cognitif futur.

15. Utilisation selon la revendication 13, dans laquelle le diagnostic a pour but de distinguer une maladie neurodégénérative et une dépression avec ou sans trouble cognitif.
